# EUROPEAN PATENT SPECIFICATION

(11) **EP 4 011 314 B1**
(45) Date of publication and mention of the grant of the patent: **25.09.2024**
(21) Application number: 20306516.4
(22) Date of filing: 08.12.2020
(51) Int. Cl.: A61B 90/00, A61B 34/20, A61B 34/10

(54) **SYSTEM AND METHOD FOR ASSISTING ORTHOPEADICS SURGERIES**
SYSTEM UND VERFAHREN ZUR UNTERSTÜTZUNG VON ORTHOPÄDISCHEN OPERATIONEN
SYSTÈME ET PROCÉDÉ D'AIDE LORS DE CHIRURGIES ORTHOPÉDIQUES

(43) Date of publication of application: 15.06.2022
(73) Proprietor: Ganymed Robotics, 75001 Paris (FR)
(72) Inventor: PETER, Loïc, 75013 Paris (FR); LOY RODAS, Nicolas, 92230 Gennevilliers (FR); DECROUEZ, Marion, 92310 Sèvres (FR)
(74) Representative: Icosa

(56) References cited:
- WO-A1-2014/122301
- WO-A1-2017/160889
- US-A1- 2008 077 158
- US-A1- 2019 380 792

## Description

### FIELD OF INVENTION

The present invention pertains to the field of image processing for orthopedic surgery. In particular, the invention relates to a computer-implemented method and system configured to predict an outcome of a planar cut performed with a planar surgical tool on a target bone of a subject during the surgery.

### BACKGROUND OF INVENTION

TKA is a surgical procedure to resurface a knee damaged by arthritis. Metal implants are used to cap the ends of the bones that form the knee-joint. During the bone preparation phase, the surgeon uses a surgical cutting tool, usually a sagittal saw to perform 5 planar cuts on the femur and one plane cut on the tibia. In the case of navigated or robotic TKA, a guidance software is used to position the cutting tool within the desired plane with respect to the bone before the cut.

Considering a guidance system used to align a planar cutting tool relative to a bone being resected according to a target plane. Such systems can guarantee up to a certain precision in the bone cut, however, several factors (patient morphology, a user's familiarity with the system, fiducial registration errors) can still affect their performance and an accurate positioning of the cutting tool may not be fully satisfied. There is no independent way to obtain a qualitative or quantitative information for verifying the accuracy of the bone resection and predicting the anatomical structure's shape after the cut, without actually performing the cut.

The present invention aims to fill this void by providing a method and system for verifying a planar cut and visualizing its future effect before the cut is performed, that can be used in conjunction with robotic and/or navigation systems.

WO 2014/122301 shows a markerless tracking apparatus and a tracking procedure for knee surgeries to navigate bone cuts. WO 2017/160889 shows a tracking system which provides an augmented view of a saw path and the planned cut of a bone.

### SUMMARY

The present invention relates to computer-implemented method for intra-operatively predicting an outcome of a planar cut performed with a planar surgical tool on a target bone of a subject, wherein said planar surgical tool comprises a planar cutting surface, said method comprising:
- receiving at least one 3D image previously acquired from at least one 3D imaging sensor; said 3D image being a 3D point cloud comprising at least one portion of the target bone and at least one portion of planar cutting surface of the surgical tool,
- segmenting the 3D image obtaining points of the 3D image belonging to the planar cutting surface of the surgical tool and points of the 3D image belonging to the target bone;
- obtaining the spatial orientation and position of an osteotomy plane by fitting a plane to the segmented points belonging to the planar cutting surface of the surgical tool;
- overlapping the osteotomy plane to the segmented points belonging to the target bone and selecting the points belonging to the portion of target bone intended to be removed with the surgical tool;
- outputting the points belonging to the portion of target bone intended to be removed with the surgical tool.

Advantageously, the present method allows to verify the future outcome of a planar cut and visualizing for the medical staff its future effect before the cut is performed, providing therefore a precious information for correcting any alignment error and the like. Furthermore, thanks to an external measurement device (i.e. 3D imaging sensor), this method allows to for an independent verification which do not rely on any kinematic or positioning data from the robotic arm or navigation system used during the surgery, thus not affected by their initial positioning errors.

According to one embodiment, the method further comprises generating a simulated 3D model of the removed bone portion using the points belonging to the portion of target bone intended to be removed with the surgical tool.

According to one embodiment, the method further comprising:
- receiving a 3D model of the bone and a preoperative planning comprising equations of the planned cutting planes in a reference frame of the 3D model of the bone,
- obtaining a planned 3D model of the removed bone portion by applying the equations of the cutting planes to the 3D model of the bone,
- comparing the simulated 3D model and said planned 3D model of the removed bone portion using 3D shape analysis and outputting the result of said comparison.

The 3D model of the bone and the simulated 3D model are 3D digital models as the planned 3D model obtained from the 3D model of the bone.

According to one embodiment, the result of the comparison is a 3D shape similarity measure or a matching error. This output of the method advantageously provides to the medical staff an information on the accuracy of positioning of the surgical tool with respect to the pre-operative planning.

According to one embodiment, the method comprises further calculating principal components of the simulated 3D model, defining from the principal components a bounding box of the removed bone portion and estimating from the bounding box a thickness of the removed bone portion. This embodiment, advantageously allows to calculate the thickness of the bone fragment (i.e. resected bone portion) before actually performing the cut.

According to one embodiment, the method further comprises comparing said estimated thickness of the removed bone portion to a planned thickness of the removed bone portion and outputting an alert whenever a deviation from the planned thickness is detected. This advantageously alert the medical staff that a correction on the positioning of the surgical tool may be performed.

According to one embodiment, the surgical tool comprises a fiducial marker to help obtaining the spatial orientation and position of the osteotomy plane.

According to one embodiment, the target bone is a femur or a tibia.

The present invention also relates to a computer program comprising instructions which, when executed by a computer, cause the computer to carry out the steps of the method according to any one of the embodiments described above.

The present invention also relates to a computer readable medium comprising instructions which, when executed by a computer, cause the computer to carry out the steps of the method according to any one of the embodiments described above.

The present invention also relates to a system for intra-operatively predicting an outcome of a planar cut performed with a planar surgical tool on a target bone of a subject, wherein the planar surgical tool comprises a planar cutting surface, said system comprising:
- at least one input adapted to receive at least one 3D image previously acquired from at least one 3D imaging sensor, said 3D image being a 3D point cloud comprising at least one portion of the target bone and at least one portion of the planar cutting surface of the surgical tool,
- at least one processor configured to:
   - segment the 3D image obtaining points of the 3D image belonging to the planar cutting surface of the surgical tool and points of the 3D image belonging to the target bone;
   - obtain the spatial orientation and position of an osteotomy plane by 3D fitting the segmented points belonging to the planar cutting surface of the surgical tool;
   - overlap the osteotomy plane to the segmented points belonging to the target bone and selecting the points belonging to the portion of target bone intended to be removed with the surgical tool;
- at least one output adapted to provide the points belonging to the portion of target bone intended to be removed with the surgical tool.

According to one embodiment, the processor is further configured to generate a simulated 3D model of the removed bone portion using the points belonging to the portion of target bone intended to be removed with the surgical tool.

According to one embodiment, the processor is further configured to:
- receive a 3D model of the bone and a preoperative planning comprising equations of the cutting planes in a reference frame of the 3D model of the bone,
- obtain a planned 3D model of the removed bone portion by applying the equations of the cutting planes to the 3D model of the bone,
- compare the simulated 3D model and said planned 3D model of the removed bone portion using 3D shape analysis and outputting the result of said comparison.

According to one embodiment, the result of the comparison is a 3D shape similarity measure or a matching error.

According to one embodiment, the processor is further configured to calculate principal components of the simulated 3D model, defining from the principal components a bounding box of the removed bone portion and estimating from the bounding box a thickness of the removed bone portion.

According to one embodiment, the processor is further configured to compare said estimated thickness of the removed bone portion to a planned thickness of the removed bone portion and outputting an alert whenever a deviation from the planned thickness is detected.

### DEFINITIONS

In the present invention, the following terms have the following meanings:
- "**bone resection thickness**" refers to the smaller dimension of the resected portion of the bone.
- "**adapted**" and "**configured**" are used in the present disclosure as broadly encompassing initial configuration, later adaptation or complementation of the present device, or any combination thereof alike, whether effected through material or software means (including firmware).
- "**processor**" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.
- "**reference frame**" refers to a coordinate system that uses one or more numbers, or coordinates, to uniquely determine the position of the points or other geometric elements on a manifold such as Euclidean space.
- "**tridimensional digital model**" refers to a three-dimensional digital (or virtual) model being a virtual object in 3 dimensions. The position and orientation of the model is known in the associated digital referential.
- "**preoperative planning**" in the context of surgery, refers to a list of actions to be performed during the different surgical phases. This surgical planning may be obtained by means of a simulation program carried out before the operation which uses a 3-dimensional digital model of the bone(s) of the patient that are the target of the surgery. In the case of a knee arthroplasty operation, for example, pre-operative planning will consist of defining each of the cutting planes and drilling axes in relation to a three-dimensional model of the femur and tibia.

### DETAILED DESCRIPTION

The following detailed description will be better understood when read in conjunction with the drawings. For the purpose of illustrating, the steps of the method are shown in the preferred embodiments. It should be understood, however that the present invention is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

Features and advantages of the invention will become apparent from the following description of embodiments of a system, this description being given merely by way of example and with reference to the appended drawings in which:
**Figure 1** is a block diagram representing the main steps of the method according to one embodiment of the present invention.
**Figure 2** is a color image of the target bone and the planar surgical tool positioned as before a planar cut is performed.
**Figure 3** is a depth image corresponding to the color image of Figure 2.
**Figure 4a** shows the point clouds belonging to both the planar cutting surface of the surgical tool and the target bone after segmentation, **4b** shows the osteotomy plane overlapping the point clouds belonging to the planar cutting surface of the surgical tool and the target bone after segmentation and **4c** the points belonging to the portion of target bone intended to be removed with the surgical tool.
**Figure 5** shows the planned 3D model of the removed bone portion and of the resected bone.
**Figure 6** shows the tibia after the performance of the planar cuts on the right and with the adequate prosthesis on the left.
**Figure 7** shows the femur after the performance of the planar cuts on the right and with the adequate prosthesis on the left.
**Figure 8** shows the bone part remove after an antero femoral cut and the thickness of the removed part measured with a sliding caliper.

### ILLUSTRATIVE EMBODIMENTS OF THE INVENTION

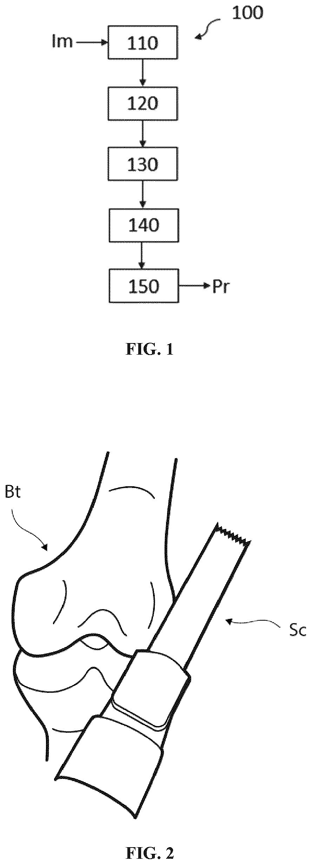

As shown in Figure 1, a first aspect of the present invention concerns a computer-implemented method 100 comprising multiple steps aiming to predict intra-operatively an outcome of at least one planar cut performed with a planar surgical tool on a target bone Bt of a subject.

Figure 2 shows a color image of the target bone Bt with the planar surgical tool positioned so that the planar cutting surface Sc has a spatial orientation and position adapted to perform the planar cut according to a preoperative planning. The surgical tool may be mounted on a kinematic chain that is configured to position the surgical tool according to the preoperative planning.

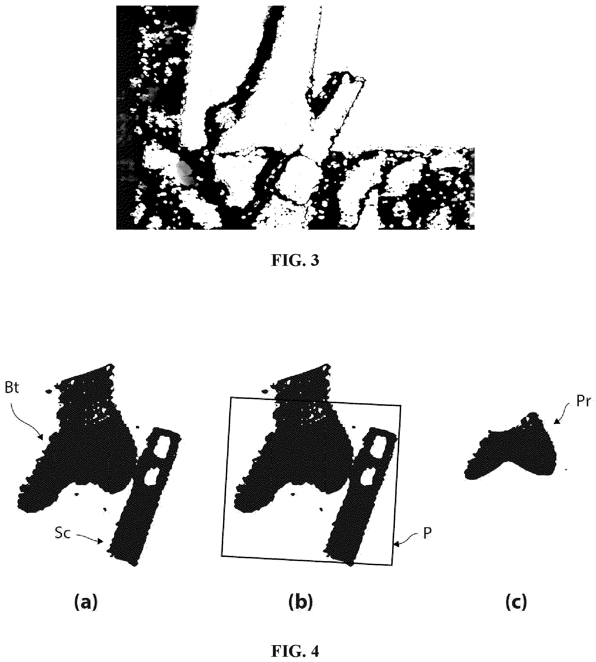

According to the embodiment in Figure 1, the first step 110 of the method consists in receiving at least one 3D image comprising at least one portion of the target bone Bt and at least one portion of planar cutting surface Sc of the surgical tool which has been acquired from at least one 3D imaging sensor before performing the cut on the target bone Bt. Figure 3 shows the depth image of the scene represented in Figure 2.

The 3D image obtained from the 3D imaging sensor comprises information of the distance between each point of the scene acquired in the image and the 3D imaging sensor. Therefore, the raw 3D image obtained by the 3D imaging sensor is also called a depth map, or depth image that may be presented under the form of a bidimensional array representing a grey level image or a RGB image, wherein the size of the array depends on the camera type and sensor dimensions.

According to one embodiment, the acquisition of the 3D image is carried out using at least two cameras to carry out an acquisition by stereovision. According to another embodiment, a projector is used to project a pattern (textured light) on the scene that will help the matching of the data streamed from the two cameras.

The use of a 3D imaging sensor advantageously allows to obtain information of the bone surface in an easy, non-invasive and fast way since one image captures all the surgical field, without coming into contact with the patient (as for palpation techniques).

The at least one 3D imaging sensor 30 may have a fixed position with respect to the target 10 in the surgical theatre and may be kinematically linked to the surgical navigation or robotic system.

The method may further comprise a pre-processing step implementing noise reduction algorithms.

According to one embodiment, the second step 120 comprises segmenting the point cloud of the 3D image so as to obtain one point cloud comprising the points belonging to the planar cutting surface of the surgical tool Sc and one point cloud comprising the point of the target bone Bt. Figure 4a shows the point clouds belonging to the planar cutting surface of the surgical tool Sc and the target bone Bt.

As shown in Figure 4b, in one embodiment, the following step 130 comprises obtaining the spatial orientation and position of an osteotomy plane P by fitting a plane to the segmented points belonging to the planar cutting surface of the surgical tool Sc.

In one embodiment, the step 140 of the method comprises selecting the points belonging to the portion of target bone intended to be removed with the surgical tool Pr by overlapping 140 the osteotomy plane P to the segmented points belonging to the target bone Bt. Figure 4c shows the point cloud points belonging to the portion of target bone intended to be removed Pr that is obtained at step 140. This advantageously allows the segmentation of the portion of target bone intended to be removed as it would be if the planar cut was performed along the suggested plane P.

In one embodiment, the method comprises the step 150 of outputting the points belonging to the portion of target bone intended to be removed with the surgical tool Pr. The output may simply be an information transferred to at least one data storage medium or database or to a digital to analogue module. Said digital to analogue module may be a display to visualize the portion of target bone intended to be removed Pr.

According to one embodiment, the method further comprises generating a simulated 3D model of the removed bone portion by rendering the points belonging to the portion of target bone intended to be removed with the surgical tool.

According to one embodiment, the method comprises retrieving from a computer readable storage medium, a server or the like a 3D model of the target bone Bt to be treated during the surgery using the surgical tool. Said 3D model is a three-dimensional virtual representation of the target bone Bt.

In one embodiment, the 3D model of the target bone Bt is generated using imaging data acquired using computed tomography or MRI systems. Other imaging techniques may be as well used such as X-rays, fluoroscopy, ultrasound or other imaging means. In this case, the three-dimensional digital model is obtained previous to the surgery.

In one embodiment, the 3D model of the target bone Bt is generated using 2D X-ray radiographies comprising the target, a statistical shape model of the target and/or the 3D image acquired intraoperatively by the 3D imaging sensor. This embodiment advantageously allows to generate a three-dimensional model even when the 3D imaging data (i.e. computed tomography or MRI) are not available.

In one embodiment, the 3D model of the target bone Bt is modified to simulate measurement noise or the presence of cartilage. Said modifications may be calculated from training data or biomechanical simulation data.

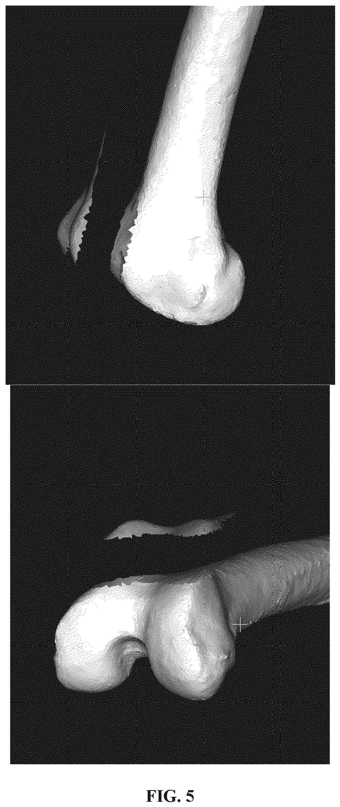

Together with the 3D model the method is configured to receive a preoperative planning which may be as well retrieved from a computer readable storage medium or a medical server. Said preoperative planning comprises the equations of the cutting planes in the same reference frame of the bone model. The equations of the cutting planes may then be used to obtain a planned 3D model of the removed bone portion by applying the cutting planes to the 3D model of the bone. Afterwards, the simulated 3D model and said planned 3D model of the removed bone portion are compared using 3D shape analysis. The result of this comparison may be a matching error or a 3D shape similarity measure. The matching error may be implemented by matching of the 3D models using ICP based methods (Iterative Closest Point) and inferring the angular deviation between the simulated cutting plane and the planned cutting plane. For the shape similarity measure shape metrics like the Hausdorff distance or the Jaccard distance may be used after the matching. Finally, the result of said comparison is provided as output. Figure 5 shows an example of visualization of the planned 3D model of the removed bone portion.

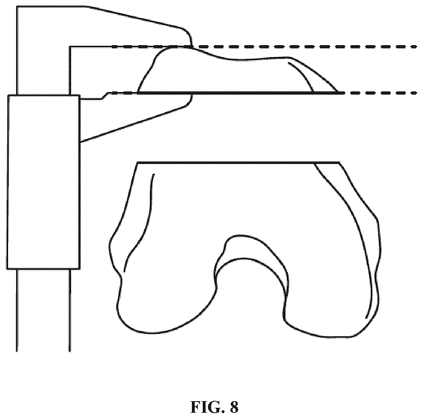

According to one embodiment, the method further comprises calculating principal components of the simulated 3D model and defining from the principal components a bounding box of the removed bone. One side of the bounding box coincides with the simulated osteotomy plane. The bounding box is the used to estimate the thickness of the removed bone portion. This step advantageously allows to calculate the thickness of the bone fragment (i.e. resected bone portion) before actually performing the cut. Figure 8 provides an illustrative representation of the thickness of the bone fragment that this method allows to calculate before the intervention and which may be measured with a sliding caliper after the surgical resection.

According to one advantageously embodiment, the method comprises comparing the estimated thickness of the removed bone portion to a planned thickness of the removed bone portion and outputting an alert whenever a deviation is detected so that the medical staff is informed that there is a discrepancy between the preoperative planning cutting planes and the actual position of the surgical tool.

In one embodiment, the surgical tool comprises a fiducial marker visible with the imaging sensor to help the estimation of the osteotomy plane. Depending on the imaging sensor, those markers can be very easy to detect in the streamed data. In the present application, infrared reflective marker may be used with a stereo vision sensor with infrared sensors or contrasted black and white marker, like Aruco markers, may be used with RGB-D cameras. Advantageously, the fiducial markers are easy to detect in the images so that it is easier to segment the points belonging to the planar surgical tool. Therefore, using the fiducial markers, the segmented points belonging to the planar cutting surface of the surgical tool contains less or no outliers and as consequence the equation of the osteotomy plane is more accurate.

Advantageously, the information obtained with the method of the present invention allows for independent verification of the preoperative planning realization.

According to one embodiment, the method comprises the visualization of the outputs of the different steps in real time. Notably, the visualization is configured to show in real time the surface of the simulated 3D model of the removed bone portion at the same time of the surface of the planned 3D model of the removed bone portion. These two models may be overlapped in a consistent manner (principal component alignment and/or registration of the 3D models) and the visualization may be configured to highlight the differences between them. These overlapped models may be displayed simultaneously to the values obtained from their comparison using 3D shape analysis so as to provide both qualitative and quantitative feedback to the user. Using such a visual feedback, the surgeon can advantageously adapt the surgical tool position to minimize the differences so that the surface of the simulated 3D model of the removed bone portion matches the surface of the planned 3D model of the removed bone portion.

The method may be used in conjunction with a navigation system. In this case, the planar surgical tool and the portion of target bone intended to be removed with the surgical tool are computed independently from the navigation system and therefore do not rely on it.

Advantageously, the method can provide an independent verification and assessment of the projected bone resection accuracy.

The method may as well be used in conjunction with a robotic system. In that case, the method does not use the knowledge of the surgical tool's position computed from the robot's kinematic and sensors. This way, the method can advantageously provide an independent verification and assessment of the projected bone resection accuracy.

The method of the present invention may be used for the target bone being the tibia or the femur.

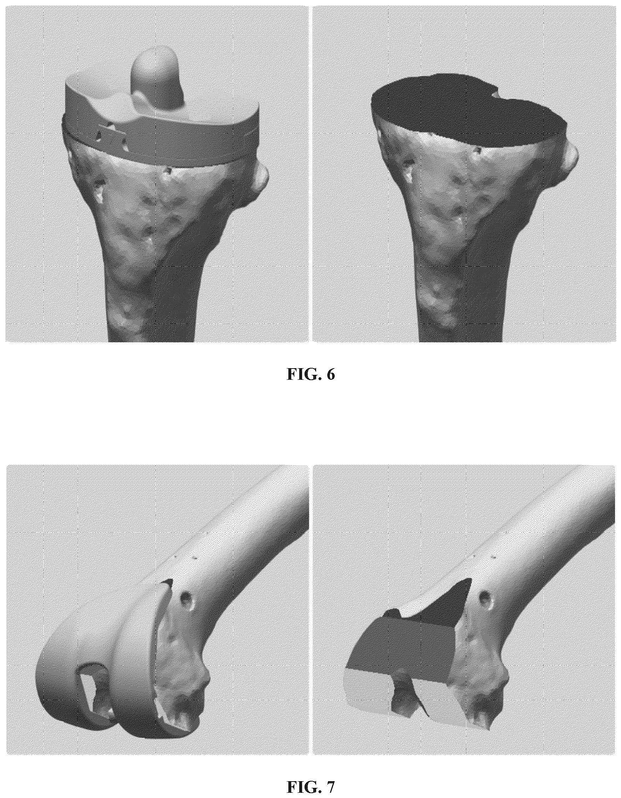

An example of the tibia after performing one cut with and without the implant is shown in Figure 6.

According to one embodiment, the steps 110 to 150 are repeated each time that the surgical tool is repositioned with respect to the target bones according to a new cutting plane comprised in the preoperative planning. Indeed, as in the example of the femur shown in Figure 7, the femur is cut according to 5 planes in order to correctly fit the implant.

The present invention also relates to the system comprising means to carry out the steps of the method described above. Notably, the system of the present invention comprises
- at least one input adapted to receive at least one 3D image previously acquired from at least one 3D imaging sensor, said 3D image being a 3D point cloud comprising at least one portion of the target bone and at least one portion of the planar cutting surface of the surgical tool,
- at least one processor configured to:
   - segmenting the points of the 3D image belonging to the planar cutting surface of surgical tool and the target bone;
   - obtaining the spatial orientation and position of an osteotomy plane by 3D fitting the segmented points belonging to the planar cutting surface of the surgical tool;
   - overlapping the osteotomy plane to the segmented points belonging to the target bone and selecting the points belonging to the portion of target bone intended to be removed with the surgical tool;
- at least one output adapted to provide the points belonging to the portion of target bone intended to be removed with the surgical tool.

In another embodiment, the system is integrated in the robot guidance software which automatically corrects the saw trajectory in order to guarantee that the cutting plane matches the surgical planning.

Embodiments disclosed herein include various operations implemented in the different steps of the method as described in this specification. As discussed above, the operations may be performed by hardware components and/or may be embodied in machine-executable instructions, which may be used to cause a general-purpose or special-purpose processor programmed with the instructions to perform the operations. Alternatively, the operations may be performed by a combination of hardware, software, and/or firmware.

The performance of one or more operations described herein may be distributed among one or more processors, not only residing within a single machine, but deployed across a number of machines. In some examples, the one or more processors or processor-implemented modules may be located in a single geographic location (e.g., within a home environment, an office environment, or a server farm). In other embodiments, the one or more processors or processor-implemented modules may be distributed across a number of geographic locations.

The present invention further comprises a computer program product for intra-operatively predicting an outcome of a planar cut, the computer program product comprising instructions which, when the program is executed by a computer, cause the computer to carry out the steps of the method according to any one of the embodiments described hereabove.

The computer program product to perform the method as described above may be written as computer programs, code segments, instructions or any combination thereof, for individually or collectively instructing or configuring the processor or computer to operate as a machine or special-purpose computer to perform the operations performed by hardware components. In one example, the computer program product includes machine code that is directly executed by a processor or a computer, such as machine code produced by a compiler. In another example, the computer program product includes higher-level code that is executed by a processor or a computer using an interpreter. Programmers of ordinary skill in the art can readily write the instructions or software based on the block diagrams and the flow charts illustrated in the drawings and the corresponding descriptions in the specification, which disclose algorithms for performing the operations of the method as described above.

The present invention further comprises a computer-readable storage medium comprising instructions which, when the program is executed by a computer, cause the computer to carry out the steps of the method according to any one of the embodiments described hereabove.

According to one embodiment, the computer-readable storage medium is a non-transitory computer-readable storage medium.

Computer programs implementing the method of the present embodiments can commonly be distributed to users on a distribution computer-readable storage medium such as, but not limited to, an SD card, an external storage device, a microchip, a flash memory device, a portable hard drive and software websites. From the distribution medium, the computer programs can be copied to a hard disk or a similar intermediate storage medium. The computer programs can be run by loading the computer instructions either from their distribution medium or their intermediate storage medium into the execution memory of the computer, configuring the computer to act in accordance with the method of this invention. All these operations are well- known to those skilled in the art of computer systems.

The instructions or software to control a processor or computer to implement the hardware components and perform the methods as described above, and any associated data, data files, and data structures, are recorded, stored, or fixed in or on one or more non-transitory computer-readable storage media. Examples of a non-transitory computer-readable storage medium include read-only memory (ROM), random-access memory (RAM), flash memory, CD- ROMs, CD-Rs, CD+ Rs, CD-RWs, CD+ RWs, DVD-ROMs, DVD-Rs, DVD+ Rs, DVD-RWs, DVD+ RWs, DVD- RAMs, BD- ROMs, BD-Rs, BD- R LTHs, BD- REs, magnetic tapes, floppy disks, magneto-optical data storage devices, optical data storage devices, hard disks, solid-state disks, and any device known to one of ordinary skill in the art that is capable of storing the instructions or software and any associated data, data files, and data structures in a non-transitory manner and providing the instructions or software and any associated data, data files, and data structures to a processor or computer so that the processor or computer can execute the instructions. In one example, the instructions or software and any associated data, data files, and data structures are distributed over network-coupled computer systems so that the instructions and software and any associated data, data files, and data structures are stored, accessed, and executed in a distributed fashion by the processor or computer.

### EXAMPLES

The present invention is further illustrated by the following practical example wherein the method and system of the present invention are applied to robotic total knee arthroplasty (TKA) procedures and especially to the preparation of the femoral surface.

At the pre-operative stage, a CT-scan of the lower limb of the patient is performed. The data is segmented to obtain a 3D model of the femur (i.e. target bone). The implant 3D model is placed on the femur 3D model using a dedicated planning software. Thus, the equations of the planar cuts are defined in the coordinate system of the femur 3D model.

During the bone preparation phase and after the femur exposition, the planar cutting tool is positioned within the planned osteotomy plane as comprised in the preoperative planning estimated by the guidance software with uncertainty in the accuracy. A depth camera images the exposed femur and the saw. The step 120 of the method is used to detect and segment the depth data belonging to the femur and the saw. Thanks to the planarity of the planar cutting surface of the saw, the osteotomy plane is estimated by fitting a 3D plane to the segmented point cloud belonging to the saw. Given the equation of the osteotomy plane, the depth data belonging to the femur is segmented by selecting the points belonging to the portion of target bone intended to be removed with the surgical tool.

Thus, the method allows to obtain a 3D representation of a simulated removed part that would be obtained if the cut were performed following the preoperative planning. The simulated bone fragment is then compared to the planned bone fragment from the preoperative stage through 3D shape analysis. The surgeon can visualize the simulated bone fragment, the simulated resected bone and the comparison with the preoperative planning on a dedicated graphical user interface displayed on a screen.

This system and method allow to compare, right before actually performing the cut (which is irreversible), whether the simulated 3D model of the removed bone portion matches the preoperative planning. The system also provides metrics to quantitively assess the similarity between the simulated and planned 3D model of the removed bone portion. This comparison does not depend on the guidance system itself (robotic arm for instance) and therefore is a separate way of checking that the surgical execution is accurate.

Surgical guidance systems also often allow to visualize the osteotomy plane before cutting (displayed on the screen or using augmented reality). However, this feature uses the exact same information that is used to position the saw in the first place, and not more. Therefore, if an error was already present in the surgical tool positioning (namely if the robot has registered that it is positioned in point A when it is, in fact, it is in point B), then the same error will be reproduced in the pre-cut verification feature.

A typical robotic system for orthopedic surgery, and TKA in particular, uses planning, initial registration and embedded sensors to direct the physical positioning of the surgical tool in space relative to the bone to be resected. Using the same information to check whether the surgical tool is properly positioned therefore does not prevent the error either.

In contrast, the present system and method advantageously allow for an independent verification relying on an external measurement device (depth camera), which do not rely on any kinematic or positioning data from the robotic or navigation system, thus not affected by their initial positioning errors.

## Claims

1. A computer-implemented method (100) for intra-operatively predicting an outcome of a planar cut to be performed with a planar surgical tool on a target bone of a subject,
wherein said planar surgical tool comprises a planar cutting surface (Sc), said method comprising:
- receiving (110) at least one 3D image (Im) previously acquired from at least one 3D imaging sensor; said 3D image (Im) being a 3D point cloud comprising at least one portion of the target bone (Bt) and at least one portion of planar cutting surface (Sc) of the surgical tool,
- segmenting (120) the 3D image (Im) obtaining points of the 3D image belonging to the planar cutting surface of the surgical tool (Sc) and points of the 3D image belonging to the target bone (Bt), said method being **characterized by**
- obtaining (130) the spatial orientation and position of an osteotomy plane (P) by fitting a plane to the segmented points belonging to the planar cutting surface of the surgical tool (Sc);
- overlapping (140) the osteotomy plane (P) to the segmented points belonging to the target bone (Bt) and selecting the points belonging to the portion of target bone intended to be removed with the surgical tool (Pr);
- outputting (150) the points belonging to the portion of target bone intended to be removed with the surgical tool (Pr).

2. The method according to claim 1, further comprising generating a simulated 3D model of the removed bone portion using the points belonging to the portion of target bone intended to be removed with the surgical tool (Pr).

3. The method according to claim **2**, further comprising:
- receiving a 3D model of the bone and a preoperative planning comprising equations of the planned cutting planes in a reference frame of the 3D model of the bone,
- obtaining a planned 3D model of the removed bone portion by applying the equations of the cutting planes to the 3D model of the bone,
- comparing the simulated 3D model and said planned 3D model of the removed bone portion using 3D shape analysis and outputting the result of said comparison.

4. The method according to claim **3**, wherein the result of the comparison is a 3D shape similarity measure or a matching error.

5. The method according to any one claims **2** to **4**, further comprising calculating principal components of the simulated 3D model, defining from the principal components a bounding box of the removed bone portion and estimating from the bounding box a thickness of the removed bone portion.

6. The method according to claim **5**, further comprising comparing said estimated thickness of the removed bone portion to a planned thickness of the removed bone portion and outputting an alert whenever a deviation from the planned thickness is detected.

7. The method according to any one claims **1** to **6**, wherein the surgical tool comprises a fiducial marker to help obtaining the spatial orientation and position of the osteotomy plane.

8. The method according to any one claims **1** to **7**, wherein the target bone is a femur or a tibia.

9. A computer program comprising instructions which, when executed by a computer, cause the computer to carry out the steps of the method according to any one of claims **1** to **8.**

10. A computer readable medium comprising instructions which, when executed by a computer, cause the computer to carry out the steps of the method according to any one of claims **1** to **8.**

11. A system for intra-operatively predicting an outcome of a planar cut performed with a planar surgical tool on a target bone of a subject, wherein the planar surgical tool comprises a planar cutting surface (Sc), said system comprising:
- at least one input adapted to receive at least one 3D image (Im) previously acquired from at least one 3D imaging sensor, said 3D image (Im) being a 3D point cloud comprising at least one portion of the target bone (Bt) and at least one portion of the planar cutting surface of the surgical tool (Sc),
- at least one processor configured to:
- segment the 3D image (Im) obtaining points of the 3D image belonging to the planar cutting surface of the surgical tool (Sc) and points of the 3D image belonging to the target bone (Bt), **characterized in that** said processor is further configured to
- obtain the spatial orientation and position of an osteotomy plane (P) by 3D fitting the segmented points belonging to the planar cutting surface of the surgical tool (Sc);
- overlap the osteotomy plane (P) to the segmented points belonging to the target bone (Bt) and selecting the points belonging to the portion of target bone intended to be removed with the surgical tool (Pr) and **in that** said system further comprises,
- at least one output adapted to provide the points belonging to the portion of target bone intended to be removed with the surgical tool (Pr).

## Patentansprüche

1. Computerimplementiertes Verfahren (100) zum intraoperativen Vorhersagen eines Ergebnisses eines ebenen Schnitts, der mit einem ebenen chirurgischen Werkzeug an einem Zielknochen eines Subjekts durchgeführt werden soll, wobei das ebene chirurgische Werkzeug eine ebene Schnittfläche (Sc) umfasst, wobei das Verfahren umfasst:
- Empfangen (110) mindestens eines 3D-Bildes (Im), das zuvor von mindestens einem 3D-Bildsensor erfasst wurde; wobei das 3D-Bild (Im) eine 3D-Punktwolke ist, die mindestens einen Abschnitt des Zielknochens (Bt) und mindestens einen Abschnitt der ebenen Schnittfläche (Sc) des chirurgischen Werkzeugs umfasst,
- Segmentieren (120) des 3D-Bildes (Im), sodass Punkte des 3D-Bildes, die zur ebenen Schnittfläche des chirurgischen Werkzeugs (Sc) gehören, und Punkte des 3D-Bildes, die zum Zielknochen (Bt) gehören, erhalten werden, wobei das Verfahren **gekennzeichnet ist durch**
- Erhalten (130) der räumlichen Ausrichtung und Position einer Osteotomieebene (P) durch Anpassen einer Ebene an die segmentierten Punkte, die zur ebenen Schnittfläche des chirurgischen Werkzeugs (Sc) gehören;
- Überlappen (140) der Osteotomieebene (P) mit den segmentierten Punkten, die zum Zielknochen (Bt) gehören, und Auswählen der Punkte, die zu dem Abschnitt des Zielknochens gehören, der mit dem chirurgischen Werkzeug (Pr) entfernt werden soll;
- Ausgeben (150) der Punkte, die zu dem Abschnitt des Zielknochens gehören, der mit dem chirurgischen Werkzeug (Pr) entfernt werden soll.

2. Verfahren nach Anspruch **1**, das weiter das Erzeugen eines simulierten 3D-Modells des entfernten Knochenabschnitts unter Verwendung der Punkte, die zu dem Abschnitt des Zielknochens gehören, der mit dem chirurgischen Werkzeug (Pr) entfernt werden soll, umfasst.

3. Verfahren nach Anspruch **2**, weiter umfassend:
- Empfangen eines 3D-Modells des Knochens und einer präoperativen Planung, die Gleichungen der geplanten Schnittebenen in einem Referenzrahmen des 3D-Modells des Knochens umfasst,
- Erhalten eines geplanten 3D-Modells des entfernten Knochenabschnitts durch Anwenden der Gleichungen der Schnittebenen auf das 3D-Modell des Knochens,
- Vergleichen des simulierten 3D-Modells und des geplanten 3D-Modells des entfernten Knochenabschnitts unter Verwendung von 3D-Formanalyse, und Ausgeben des Ergebnisses des Vergleichs.

4. Verfahren nach Anspruch **3**, wobei das Ergebnis des Vergleichs ein 3D-Formähnlichkeitsmaß oder ein Übereinstimmungsfehler ist.

5. Verfahren nach einem der Ansprüche **2** bis **4**, das weiter das Berechnen von Hauptkomponenten des simulierten 3D-Modells, Definieren eines Begrenzungskästchens des entfernten Knochenabschnitts anhand der Hauptkomponenten, und Schätzen einer Dicke des entfernten Knochenabschnitts anhand des Begrenzungskästchens umfasst.

6. Verfahren nach Anspruch **5**, das weiter das Vergleichen der geschätzten Dicke des entfernten Knochenabschnitts mit einer geplanten Dicke des entfernten Knochenabschnitts und Ausgeben eines Alarms umfasst, wenn eine Abweichung von der geplanten Dicke erkannt wird.

7. Verfahren nach einem der Ansprüche **1** bis **6**, wobei das chirurgische Werkzeug eine Passermarke umfasst, um zu helfen, die räumliche Ausrichtung und Position der Osteotomieebene zu erhalten.

8. Verfahren nach einem der Ansprüche **1** bis **7**, wobei der Zielknochen ein Oberschenkelknochen oder ein Unterschenkelknochen ist.

9. Computerprogramm, das Anweisungen umfasst, die, wenn sie von einem Computer ausgeführt werden, den Computer dazu bringen, die Schritte des Verfahrens nach einem der Ansprüche **1** bis **8** auszuführen.

10. Computerlesbares Medium, das Anweisungen umfasst, die, wenn sie von einem Computer ausgeführt werden, den Computer dazu bringen, die Schritte des Verfahrens nach einem der Ansprüche **1** bis **8** auszuführen.

11. System zum intraoperativen Vorhersagen eines Ergebnisses eines ebenen Schnitts, der mit einem ebenen chirurgischen Werkzeug an einem Zielknochen eines Subjekts durchgeführt wird, wobei das ebene chirurgische Werkzeug eine ebene Schnittfläche (Sc) umfasst, wobei das System umfasst:
- mindestens einen Eingang, der geeignet ist, mindestens ein 3D-Bild (Im) zu empfangen, das zuvor von mindestens einem 3D-Bildsensor erfasst wurde, wobei das 3D-Bild (Im) eine 3D-Punktwolke ist, die mindestens einen Abschnitt des Zielknochens (Bt) und mindestens einen Abschnitt der ebenen Schnittfläche des chirurgischen Werkzeugs (Sc) umfasst,
- mindestens einen Prozessor, der so konfiguriert ist, dass er:
-- das 3D-Bild (Im) segmentiert, sodass Punkte des 3D-Bildes, die zur ebenen Schnittfläche des chirurgischen Werkzeugs (Sc) gehören, und Punkte des 3D-Bildes, die zum Zielknochen (Bt) gehören, erhalten werden, **dadurch gekennzeichnet, dass** der Prozessor weiter so konfiguriert ist, dass er
-- durch 3D-Anpassen der segmentierten Punkte, die zur ebenen Schnittfläche des chirurgischen Werkzeugs (Sc) gehören, die räumliche Ausrichtung und Position einer Osteotomieebene (P) erhält;
-- die Osteotomieebene (P) mit den segmentierten Punkten, die zum Zielknochen (Bt) gehören, überlappt und die Punkte auswählt, die zu dem Abschnitt des Zielknochens gehören, der mit dem chirurgischen Werkzeug (Pr) entfernt werden soll, und dadurch, dass das System weiter umfasst
- mindestens einen Ausgang, der geeignet ist, die Punkte bereitzustellen, die zu dem Abschnitt des Zielknochens gehören, der mit dem chirurgischen Werkzeug (Pr) entfernt werden soll.

## Revendications

1. Un procédé mis en oeuvre par ordinateur (100) pour prédire de manière intra-opératoire un résultat d'une coupe plane à réaliser avec un outil chirurgical plan sur un os cible d'un sujet, ledit outil chirurgical plan comprenant une surface de coupe plane (Sc), ledit procédé comprenant :
- la réception (110) d'au moins une image 3D (Im) précédemment acquise à partir d'au moins un capteur d'imagerie 3D ; ladite image 3D (Im) étant un nuage de points en 3D comprenant au moins une partie de l'os cible (Bt) et au moins une partie de la surface de coupe plane (Sc) de l'outil chirurgical,
- la segmentation (120) de l'image 3D (Im) en obtenant des points de l'image 3D appartenant à la surface de coupe plane de l'outil chirurgical (Sc) et des points de l'image 3D appartenant à l'os cible (Bt),
ledit procédé étant **caractérisé en ce que**
- l'obtention (130) de l'orientation spatiale et de la position d'un plan d'ostéotomie (P) en ajustant un plan aux points segmentés appartenant à la surface de coupe plane de l'outil chirurgical (Sc) ;
- la superposition (140) du plan d'ostéotomie (P) aux points segmentés appartenant à l'os cible (Bt) et la sélection des points appartenant à la partie d'os cible destinée à être retirée avec l'outil chirurgical (Pr) ;
- la sortie (150) des points appartenant à la partie d'os cible destinée à être retirée avec l'outil chirurgical (Pr).

2. Le procédé selon la revendication 1, comprenant en outre la génération d'un modèle 3D simulé de la partie d'os retirée en utilisant les points appartenant à la partie d'os cible destinée à être retirée avec l'outil chirurgical (Pr).

3. Le procédé selon la revendication 2, comprenant en outre :
- la réception d'un modèle 3D de l'os et d'une planification préopératoire comprenant des équations des plans de coupe prévus dans un référentiel du modèle 3D de l'os,
- l'obtention d'un modèle 3D prévu de la partie d'os retirée en appliquant les équations des plans de coupe au modèle 3D de l'os,
- la comparaison du modèle 3D simulé et dudit modèle 3D prévu de la partie d'os retirée en utilisant une analyse de forme en 3D et la sortie du résultat de ladite comparaison.

4. Le procédé selon la revendication 3, dans lequel le résultat de la comparaison est une mesure de similarité de forme en 3D ou une erreur de correspondance.

5. Le procédé selon l'une quelconque des revendications 2 à 4, comprenant en outre le calcul des composants principaux du modèle 3D simulé, la définition à partir des composants principaux d'une boîte englobante de la partie d'os retirée et l'estimation à partir de la boîte englobante d'une épaisseur de la partie d'os retirée.

6. Le procédé selon la revendication 5, comprenant en outre la comparaison de ladite épaisseur estimée de la partie d'os retirée à une épaisseur planifiée de la partie d'os retirée et la sortie d'une alerte chaque fois qu'une déviation par rapport à l'épaisseur planifiée est détectée.

7. Le procédé selon l'une quelconque des revendications 1 à 6, dans lequel l'outil chirurgical comprend un repère fiducial pour aider à obtenir l'orientation spatiale et la position du plan d'ostéotomie.

8. Le procédé selon l'une quelconque des revendications 1 à 7, dans lequel l'os cible est un fémur ou un tibia.

9. Un programme informatique comprenant des instructions qui, lorsqu'elles sont exécutées par un ordinateur, amènent l'ordinateur à effectuer les étapes du procédé selon l'une quelconque des revendications 1 à 8.

10. Un support lisible par ordinateur comprenant des instructions qui, lorsqu'elles sont exécutées par un ordinateur, amènent l'ordinateur à effectuer les étapes du procédé selon l'une quelconque des revendications 1 à 8.

11. Un système pour prédire de manière intra-opératoire un résultat d'une coupe plane réalisée avec un outil chirurgical plan sur un os cible d'un sujet, ledit outil chirurgical plan comprenant une surface de coupe plane (Sc), ledit système comprenant :
- au moins une entrée conçue pour recevoir au moins une image 3D (Im) préalablement acquise à partir d'au moins un capteur d'imagerie 3D, ladite image 3D (Im) étant un nuage de points en 3D comprenant au moins une partie de l'os cible (Bt) et au moins une partie de la surface de coupe plane de l'outil chirurgical (Sc),
- au moins un processeur configuré pour :
∘ segmenter l'image 3D (Im) en obtenant des points de l'image 3D appartenant à la surface de coupe plane de l'outil chirurgical (Sc) et des points de l'image 3D appartenant à l'os cible (Bt) ;
**caractérisé en ce que** ledit processeur est en outre configuré pour
- obtenir l'orientation spatiale et la position d'un plan d'ostéotomie (P) en ajustant en 3D les points segmentés appartenant à la surface de coupe plane de l'outil chirurgical (Sc) ;
- superposer le plan d'ostéotomie (P) aux points segmentés appartenant à l'os cible (Bt) et sélectionner les points appartenant à la partie d'os cible destinée à être retirée avec l'outil chirurgical (Pr) ;
et **en ce que** ledit système comprend en outre :
- - au moins une sortie conçue pour fournir les points appartenant à la partie d'os cible destinée à être retirée avec l'outil chirurgical (Pr).
